(19) Europäisches Patentamt
European Patent Office
Office européen des brevets

(11) Publication number : **0 518 414 B1**

(12) # EUROPEAN PATENT SPECIFICATION

(45) Date of publication of patent specification : **08.06.94 Bulletin 94/23**

(51) Int. Cl.$^5$ : **C07D 335/16,** C07F 9/24, A61K 31/38

(21) Application number : **92201587.0**

(22) Date of filing : **03.06.92**

(54) **Thioxanthenone antitumor agents.**

(30) Priority : **10.06.91 US 713173**
**13.02.92 US 835159**

(43) Date of publication of application : **16.12.92 Bulletin 92/51**

(45) Publication of the grant of the patent : **08.06.94 Bulletin 94/23**

(84) Designated Contracting States : **AT BE CH DE DK ES FR GB GR IT LI LU MC NL PT SE**

(56) References cited :
**US-A- 3 745 172**
**US-A- 4 539 412**
**JOURNAL OF MEDICINAL CHEMISTRY, vol. 6, no. 2, March 1963, Washington, DC, US, pages 185 - 191; E.J. BLANZ ET AL.: 'A systematic investigation of thioxanthen-9-ones and analogues as potential antitumour agents'**
**JOURNAL OF MEDICINAL CHEMISTRY. vol. 31, no. 4, April 1988, Washington, DC, US, pages 707 - 712; B.D. PALMER ET AL.: 'Potential antitumour agents. 54. Chromophore requirements for in vivo antitumour activity among the general class of linear tricyclic carboxamides'**
**JOURNAL OF PHARMACEUTICAL SCIENCES. vol. 54, no. 11, November 1965, Washington, DC, US, pages 1672 - 1673; I. NABIH ET AL.: 'Chlorination and condensation reactions at the 4-methyl group of lucanthone and oxalucanthone'**
**patent abstracts, vol. 21, february 1993, derwent publications, London, GB**

(73) Proprietor : **STERLING WINTHROP INC.**
**90 Park Avenue**
**New York, NY 10016 (US)**

(72) Inventor : **Miller, Theodore Charles**
**c/o Sterling Winthrop Inc,**
**81 Columbia Turnpike**
**Rensselaer, NY 12144 (US)**
Inventor : **Collins, Joseph Charles**
**c/o Sterling Winthrop Inc,**
**81 Columbia Turnpike**
**Rensselaer, NY 12144 (US)**
Inventor : **Wentland, Mark Philip**
**c/o Sterling Winthrop Inc,**
**81 Columbia Turnpike**
**Rensselaer, NY 12144 (US)**
Inventor : **Perni, Robert Bruno**
**c/o Sterling Winthrop Inc,**
**81 Columbia Turnpike**
**Rensselaer, NY 12144 (US)**
Inventor : **Corbett, Thomas Hughes**
**c/o Sterling Winthrop Inc,**
**81 Columbia Turnpike**
**Rensselaer, NY 12144 (US)**
Inventor : **Mattes, Kenneth Charles, c/o**
**Eastman Kodak Company**
**Patent Department,**
**343 State Street**
**Rochester, NY 14650-2201 (US)**

(74) Representative : **Haile, Helen Cynthia et al**
**Kodak Limited**
**Patent Department**
**Headstone Drive**
**Harrow, Middlesex HA1 4TY (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

The present invention relates to novel 1-[[(dialkylamino)-alkyl]amino]-4-substituted-thioxanthen-9-ones, to pharmaceutical compositions containing the thioxanthenones, to methods of treating tumors with the thioxanthenones and to methods of treating cancer in mammals with the compositions containing the thioxanthenones.

Nabih and Elsheikh [J. Pharm. Sci. 54, 1672-1673 (1965)] disclose 1-[[2-(diethylamino)ethyl]amino]-4-[(diethylamino)-methyl]thioxanthen-9-one. No utility was demonstrated for the compound.

Collins and Rosi U.S. Patent 3,745,172 disclose as an intermediate in the synthesis of antifungal and antibacterial agents:

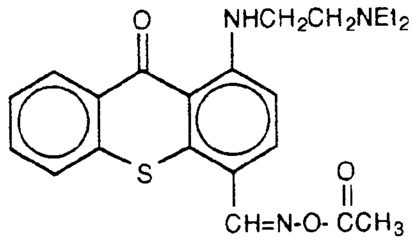

and as an anthelmintic and antibacterial agent:

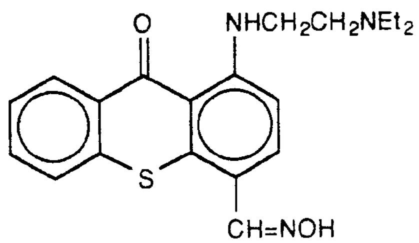

Rosi and Peruzotti U. S. Patent 3,312,598 disclose 1-[[2-(diethylamino)ethyl]amino]-9-oxo-9H-thioxanthene-4-carboxylic acid as a by-product of fermentation having no disclosed utility.

Blanz and French [J. Med. Chem. 6, 185-191 (1963)] disclose the synthesis of a series of thioxanthenones related to lucanthone and the results of the testing of the compounds against a leukaemia and two solid tumors. Among the compounds disclosed are

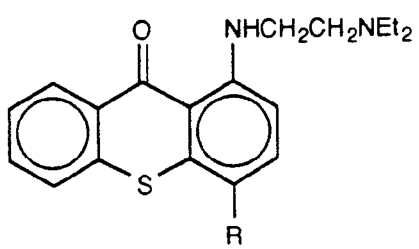

wherein R is methyl, methoxy, and ethoxy.

Yarinsky and Freele [Journal of Tropical Medicine and Hygiene 73, 23-27 (1970)] disclose

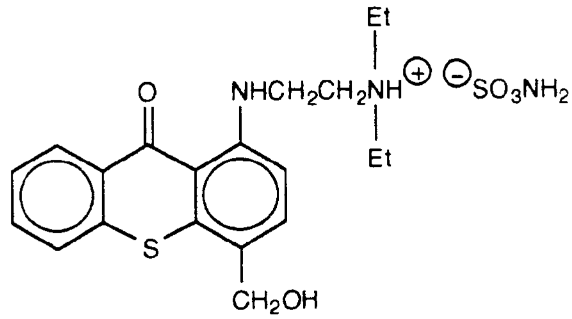

as an antischistosomal agent.

Palmer et al.[J. Med. Chem. 31, 707-712 (1988)] disclose N-[2-(dimethylamino)ethyl]-9-oxo-9H-thioxanthene-4-carboxamide monohydrochloride which was tested in vitro versus murine leukaemia (L1210) and in vivo versus P388 leukaemia cells and was found to be "unlikely to be worth pursuing" as a potential antitumor agent.

The present invention relates to compounds of the formula I

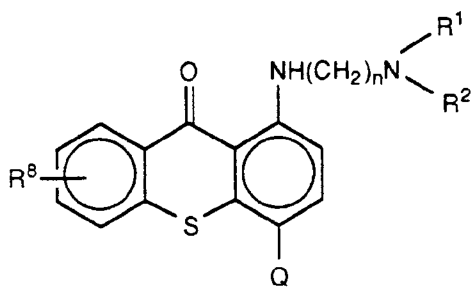

I

or acid addition salt or solvate thereof wherein n is two or three, preferably two; $R^1$ and $R^2$ are independently lower-alkyl, preferably both are ethyl; Q is a residue chosen from the group consisting of $-CH_2NHR^3$, $-CH_2N(R^4)SO_2R^7$, $-CH_2NHCHO$, -CH=N-Ar, $-C(O)NR^5R^6$, $CH_2N(R^4)C(O)R^7$, $CH_2N(C_2H_5)CHO$, and $CH_2N(R^4)P(O)$(O-lower-alkyl); $R^3$ is hydrogen or lower-alkyl; $R^4$ is hydrogen or lower-alkyl; $R^5$ is hydrogen, lower-alkyl, or Ar, preferably hydrogen; $R^6$ is hydrogen or lower-alkyl, preferably hydrogen; $R^7$ is lower-alkyl, preferably methyl, or Ar; $R^8$ is hydrogen, lower-alkyl, lower-alkoxy or halogen; and Ar is phenyl or phenyl substituted with methyl, methoxy, halogen or nitro. Ar is preferably phenyl. The compounds are useful for the treatment of tumors in mammals.

Lower-alkyl as used herein describes linear, branched or cyclic hydrocarbons containing four or fewer carbon atoms. Halogen describes bromine, chlorine or fluorine.

The invention further relates to compositions for treating tumors and cancer in mammals which comprise compounds of formula I together with pharmaceutically acceptable excipients or diluents.

The invention further relates to a method of use of a compound of formula I for treating tumors in mammals which comprises administering to the mammal a compound of formula I.

The invention further relates to a method of use of a compound of formula I for treating cancer in a mammal which comprises administering to the mammal a composition of a compound of formula I together with pharmaceutically acceptable excipients or diluents.

The synthesis of compounds of the invention may be outlined as shown in Schemes A and B:

**SCHEME A**

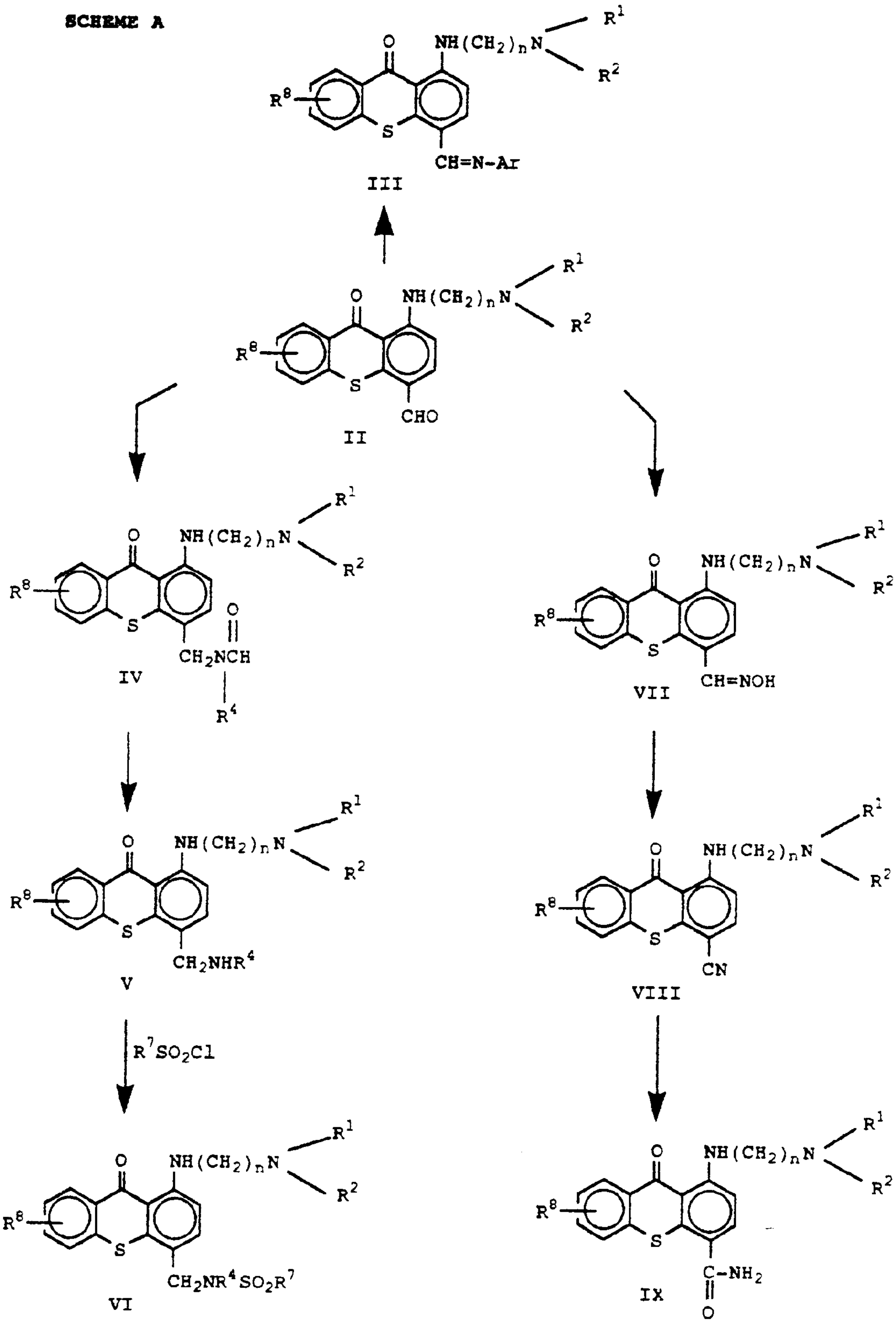


III

II

IV

VII

V

VIII

VI

IX

**Scheme B**

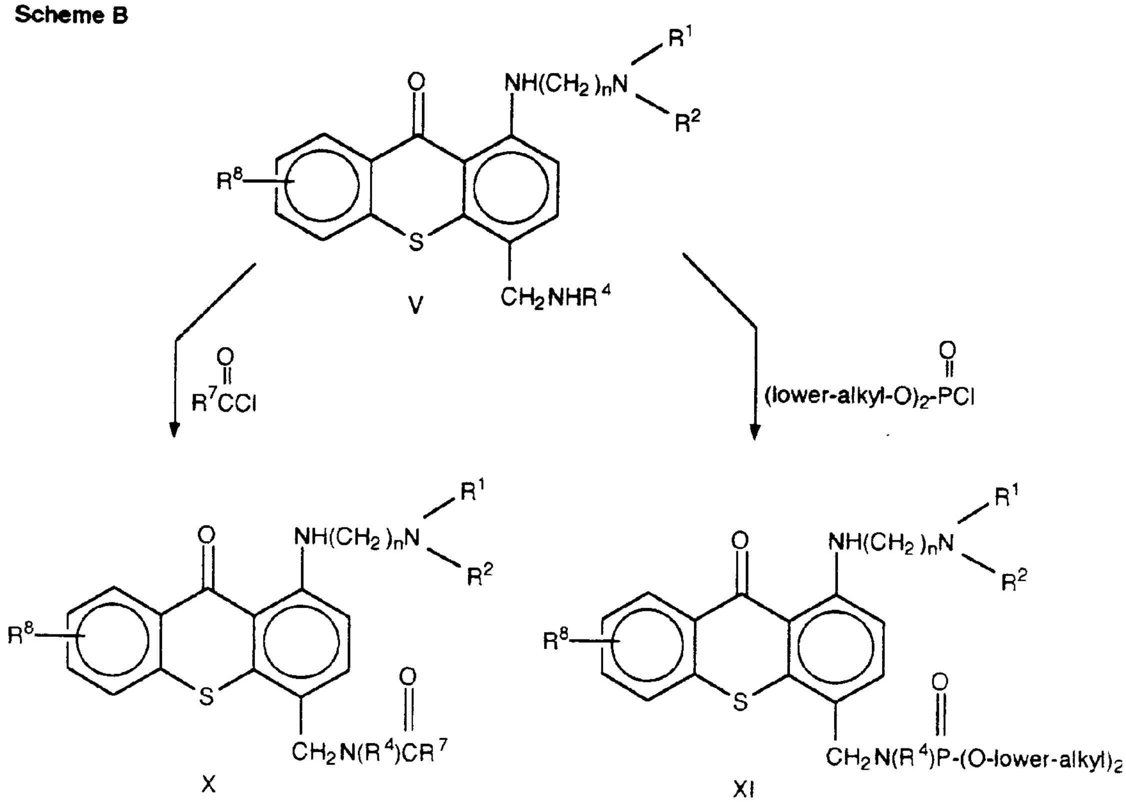


The compounds of formula III (formula I wherein Q is -CH=N-Ar) may be synthesized by heating an aldehyde of formula II together with about one equivalent of the appropriate aniline in an inert, azeotroping solvent, preferably xylene or toluene at reflux.

The compounds of formula IV may be prepared from an aldehyde of formula II by heating at 150° to 185°C in the presence of about 10 equivalents of formic acid in formamide or N-alkylformamide as a solvent. The conditions are well known in the art for the Leuckart reaction. The compounds of formula V are then obtained by acid hydrolysis of the formamide.

The compounds of formula VI (formula I wherein Q is $-CH_2N(R^4)SO_2R^7$) may be synthesised by sulfonylation of the amine V with a slight excess of a lower-alkylsulfonyl, or arylsulfonyl chloride in a suitable solvent, preferably pyridine, at 0° to 50°C.

Alternatively, the compounds of formula VI, wherein $R^4$ is lower-alkyl, may be synthesized by sulfonylation of the amine V, wherein $R^4$ is hydrogen, as described above, followed by treatment of the resulting sulfonamide VI, $R^4$ is hydrogen, with an excess of a base, preferably sodium hydride, in a suitable solvent, such as tetrahydrofuran, followed by treatment with an excess of an appropriate lower-alkyl halide at a temperature in the range of about 0°C up to the boiling point of the solvent used.

The carboxamides of formula IX may be synthesized by reacting the aldehyde II with a 5-6 fold excess of hydroxylamine hydrochloride in pyridine, optionally containing a cosolvent, followed by dehydration of the oxime (VII) by treating with an excess of acetic anhydride and heating in an inert, high-boiling solvent such as xylene, and, finally, partial hydrolysis of the nitrile (VIII) in concentrated sulfuric acid. In the cases where it is desired that $R^5$ and $R^6$ be other than hydrogen, the amide IX may be further hydrolyzed to the corresponding acid in 16% alcoholic KOH and the acid may be condensed with the appropriate amine using procedures well known in the art.

The compounds of formula X (formula I wherein Q is $CH_2N(R^4)C(O)R^7$) may be synthesized by acylation of the amine V with an excess of a lower-alkyl acid chloride, or aryl acid chloride in a suitable solvent, preferably pyridine, optionally in the presence of a cosolvent, preferably dichloromethane, at a temperature in the range from about 0°C to about 50°C.

The compounds of formula XI (formula I wherein Q is $CH_2N(R^4)P(O)(O\text{-lower-alkyl})_2$) may be synthesized by treatment of the amine V with an excess of an appropriate di-lower-alkyl phosphorochloridate in a suitable solvent, such as dichloromethane, in the presence of an excess of a base, preferably triethylamine, at a temperature in the range of from about 0°C up to about 50°C.

The aldehyde II is available by the method disclosed in U.S. Patent 3,294,803 and by $MnO_2$ oxidation of the alcohol obtained by the method of U.S. Patent 3,711,512.

The compounds of formula I are useful both in the free base form and in the form of acid-addition salts, and both forms are within the purview of the invention. The acid-addition salts are in some cases a more convenient form for use, and in practice the use of the salt form inherently amounts to the use of the base form. The acids which can be used to prepare the acid-addition salts include preferably those which produce, when combined with the free base, medicinally acceptable salts, that is, salts whose anions are relatively innocuous to the animal organism in medicinal doses of the salts so that the beneficial properties inherent in the free base are not vitiated by side effects ascribable to the anions. In practising the present invention it is convenient to form the hydrochloride, fumarate, toluenesulfonate, methanesulfonate, or maleate salts. However, other appropriate medicinally acceptable salts within the scope of the invention are those derived from other mineral acids and organic acids. The acid-addition salts of the basic compounds are prepared either by dissolving the free base in aqueous alcohol solution containing the appropriate acid and isolating the salt by evaporating the solution, or by reacting the free base and an acid in an organic solvent, in which case the salt separates directly, is precipitated with a second organic solvent, or can be obtained by concentration of the solution. Although medicinally acceptable salts of the basic compounds are preferred, all acid-addition salts are within the scope of the present invention. All acid-addition salts are useful as sources of the free base form even if the particular salt per se is desired only as an intermediate product, as, for example, when the salt is formed only for purposes of purification or identification, or when it is used as an intermediate in preparing a medicinally acceptable salt by ion exchange procedures.

The structures of the compounds of the invention were established by the mode of synthesis, elemental analysis, and infrared, ultraviolet and nuclear magnetic resonance spectroscopy. The course of the reactions and the identity and homogeneity of the products were assessed by thin layer chromatography (TLC) or gas-liquid chromatography (GLC). The melting points are given in degrees C and are uncorrected. The starting materials are either commercially available or may be prepared by procedures well known in the art.

The invention will now be more particularly described with reference to the following examples which in no way limit the scope of the invention.

Example 1

1-[[2-(Diethylamino)ethyl]amino]-4-(N-phenylformimidoyl) thioxanthen-9-one

(I: $R^1$=$R^2$=Et; Q=CH=N-$C_6H_5$; $R^8$=H; n=2) (III;Ar=Ph)

A mixture of 17.7 g (50 mmol) of 1-[[2-(diethylamino)-ethyl]amino]thioxanthen-4-carboxyaldehyde and 15.1 g (150 mmol) of aniline in 100 ml of toluene was refluxed for 8 hours with a Dean-Stark trap. TLC on alumina with chloroform/hexane/isopropylamine 10:10:2 indicated incomplete reaction. The toluene was distilled off, 25 ml of aniline was added and the mixture refluxed for 4 hours. 50 ml of xylene was added and the reaction refluxed again for 3 hours. The solvent and excess aniline were removed in vacuo and the residue recrystallized from benzene to yield 19.9 g of crude product. This was recrystallized from approximately 1.5 l hexane to yield 15.8 g (86%) of product, m.p. 125-126°.

Example 2

N-[[1-[[2-(Diethylamino)ethyl]amino]-9-oxothioxanthen-4-yl]methyl]formamide

(I: $R^1$=$R^2$=Et; Q=$CH_2$NHCHO; $R^8$=H; n=2) (IV;$R^4$=H)

A solution of 35.4 g (0.1 mol) of 1-[[2-(diethylamino)-ethyl]amino]thioxanthen-4-carboxyaldehyde, 420 ml of formamide and 50 ml (1 mol) of formic acid was heated at 160° for 1 hour. The reaction was cooled, poured into 2 l of water and made basic with about 50 ml of 35% sodium hydroxide solution. The gummy precipitate was filtered off and dried in vacuo. The dried precipitate was dissolved in about 1.5 l of hot ethyl acetate, treated with charcoal, and crystallized by cooling. The product was filtered off, washed with ethyl acetate and dried to provide 29.0 g (75%) of product, m.p. 154-155°.

Example 3

N-[[1-[[2-(Diethylamino)ethyl]amino]-9-oxothioxanthen-4-yl]methyl]-N-methylformamide

(IV: $R^1$=$R^2$=Et;$R^4$=Me;$R^8$=H; n=2)

By a procedure analogous to that of Example 2, 24.6 g of the N-methylformamide was prepared from 35.4 g (0.1 mol) of 1-[[2-(diethylamino)ethyl]amino]thioxanthen-4-carboxyaldehyde, 394 g of N-methylformamide and 50 ml of formic acid. The product was recrystallized from 150 ml of acetone to a m.p. of 127-130°.

Example 4

4-(Aminomethyl)-1-[[2-(diethylamino)ethyl]amino]thioxanthen-9-one

(I: $R^1$=$R^2$=Et; Q=$CH_2NH_2$; $R^8$=H; n=2)(V;$R^4$=H)

A solution of 24.4 g (64 mmol) of the formamide of Example 2 in 240 ml of 2N hydrochloric acid was heated on a steam bath for 1 hour. The reaction was cooled to room temperature, made basic with 35% aqueous sodium hydroxide, and the resulting yellow precipitate collected by filtration. The product was dissolved in benzene, treated with charcoal, dried with magnesium sulfate, filtered and azeotroped to remove traces of water. The dried residue was crystallized from methanol and isopropanol by the addition of ethereal hydrogen chloride. The resulting solid was recrystallized in several crops from methanol to yield 10.6 g of product, m.p. 270-272°, as the dihydrochloride salt.

Example 5

1-[[2-(Diethylamino)ethyl]amino]-4-[(methylamino)-methyl]thioxanthen-9-one

(I: $R^1$=$R^2$=Et; Q=$CH_2NHCH_3$; $R^8$=H; n=2)(V;$R^4$=Me)

By a process precisely analogous to that of Example 4, 10.5 g of the methylamine was obtained as the dihydrochloride hemihydrate from 14.6 g (37 mmol) of the N-methylformamide of Example 3 and 150 ml of 2N hydrochloric acid. The product melted at 241-243°.

Example 6

N-[[1-[[2-(Diethylamino)ethyl]amino]-9-oxothioxanthen-4-yl]methyl]methanesulfonamide

(I: $R^1$=$R^2$=Et; Q=$CH_2NHSO_2CH_3$; $R^8$=H; n=2)(VI;$R^4$=H;$R^7$=Me)

A solution of 10.65 g (30 mmol) of the free base of the amine of Example 4 in 100 ml of pyridine was cooled in an ice bath and 4 g (35 mmol) of methanesulfonylchloride was added in one portion. The mixture was stirred for 2 hours at room temperature and poured into 750 ml of water containing 2 g of sodium hydroxide. The dark yellow precipitate was collected, washed with water and dried in vacuo overnight. A second crop was obtained by adding excess sodium hydroxide to the filtrate and filtering the resulting solid. The combined precipitates after drying were recrystallized from benzene to yield 6.4 g of the methanesulfonamide, m.p. 169-170°.

Example 7

1-[[2′-(Diethylamino)ethyl]amino]-9-oxothioxanthene-4-carboxamide

(I: $R^1$=$R^2$=Et; Q=$CONH_2$; $R^8$=H; n=2)(IX)

A suspension of 74g (0.23 mol) of 1-[[2-(diethylamino)-ethyl]amino]thioxanthen-4-carboxaldehyde and 74 g (1.06 mol) of hydroxylamine hydrochloride in 400 ml of pyridine and 400 ml of ethanol was refluxed 0.5 hour and 70 ml of water was added to provide a homogeneous solution. The solution was heated for a further 2 hours and allowed to sit at room temperature 14 hours. The resulting crystalline oxime (VII) was filtered off to provide a quantitative yield, m.p. 215-218°.

One hundred twenty-three grams of the oxime was heated briefly on a steambath in 180 ml of acetic anhydride to achieve solution. The solution was cooled, 100 ml of 1.8 M HCl in ether was added and the resulting suspension was diluted with 500 ml of ether. The suspension was allowed to sit 14 hours at 0° and filtered. The residue (123g, m.p. 109-112°) was slurried in 250 mL of xylene and refluxed 20 min. The mixture was cooled and 71.3g of the nitrile (VIII) was filtered off, m.p. 265°.

Ten grams of the nitrile was stirred in 200 ml of conc. $H_2SO_4$ at room temperature for 3 days. The reaction was neutralized with conc. $NH_4OH$ and the residue filtered off. The residue was digested in warm EtOAc/EtOH, filtered and the product crystallized from the chilled solution, m.p. 241-243°. It was dissolved in ethanol and one equivalent of HCl in ethanol was added. Six grams of the amide hydrochloride was obtained, m.p. 271-272°.

Example 8

N-[[1-[[2-(Diethylamino)ethyl)amino]-9-oxothioxanthen-4-yl]methyl]N-methylmethanesulfonamide

(I: $R^1$=$R^2$=Et; Q=$CH_2N(CH_3)SO_2CH_3$; $R^8$=H; n=2)(VI;$R^4$=Me;$R^7$=Me)

A solution of 1.5 g (3.5 mmol) of the methanesulfonamide of Example 6 in THF (60 ml) was cooled to 0°C in an ice-bath and NaH 0.16 g (4.0 mmol) was added. The reaction mixture was warmed to room temperature, stirred for 10 minutes, then methyl iodide 0.25 mL (4.0 mmol) was added. The reaction mixture was stirred at room temperature for 24 hours and the solvent was removed in vacuo. The residue was purified by column chromatography on silica eluting with chloroform (100%) then 1% isopropylamine/chloroform to afford 1.15 g (74%) of the N-methylmethanesulfonamide as a yellow powder, m.p. 175-177°C.

Example 9

N-[[1-[[2-(Diethylamino)ethyl]amino]-9-oxothioxanthen-4-yl]methyl]phenylsulfonamide

(I: $R^1$=$R^2$=Et; Q=$CH_2NHSO_2Ph$; $R^8$=H; n=2)(VI;$R^4$=H;$R^7$=Ph)

Following a procedure substantially similar to that described in Example 6, 2.4 g (57%) of the phenylsulfonamide was obtained as the methanesulfonic acid salt from 2.54 g (7.15 mmol) of the free base of the amine of Example 4, pyridine (50 ml) and benzenesulfonyl chloride (1.1 ml, 8.62 mmol), followed by treatment of the residue thus obtained with methanesulfonic acid in methanol. The product was recrystallized from ethanol.

Example 10

N-[[1-[[2-(Diethylamino)ethyl]amino]-9-oxothioxanthen-4-yl]methyl]acetamide

(I: $R^1$=$R^2$=Et; Q=$CH_2NHC(O)CH_3$; $R^8$=H; n=2)(X;$R^4$=H;$R^7$=Me)

Following a procedure substantially similar to that described in Example 6, 2.3 g (52%) of the acetamide was obtained as an orange solid from 4.15 g (11.7 mmol) of the free base of the amine of Example 4, pyridine (60 ml) and acetyl chloride (0.82 ml, 11.53 mmol). The product was recrystallized from acetone and melted at 182-183°C.

Example 11

N-[[1-[[2-(Diethylamino)ethyl]amino]-9-oxothioxanthen-4-yl]methyl]benzamide

(I: $R^1$=$R^2$=Et; Q=$CH_2NHC(O)Ph$; $R^8$=H; n=2)(X;$R^4$=H;$R^7$=Ph)

Following a procedure substantially similar to that described in Example 6, 1.02 g (68%) of the benzamide was obtained as a yellow powder from 1.17 g (3.29 mmol) of the free base of the amine of Example 4, pyridine (25 ml) and benzoyl chloride (0.42 ml, 3.62 mmol). The product was purified by column chromatography on silica eluting with chloroform (100%) to 1% isopropylamine/chloroform, followed by recrystallization from ethyl acetate. The product melted at 161-163°C.

Example 12

N-[[1-[[2-(Diethylamino)ethyl]amino]-9-oxothioxanthen-4-yl]diethyl phosphoramide

(I: $R^1$=$R^2$=Et; Q=$CH_2NHP(O)(OEt)_2$; $R^8$=H; n=2)(XI;$R^4$=H)

A solution of 2.28 g (6.41 mmol) of the free base of the amine of Example 4, $CH_2Cl_2$ (50 ml), and triethylamine (2 ml) at 0°C was treated with diethyl phosphorochloridate (1.0 ml, 6.9 mmol). The reaction mixture was stirred at 0°C for 2 hours, then at room temperature for 1 hour. The solvent was removed in vacuo and the residue was purified by column chromatography on silica eluting with ethyl acetate (100%),then 5% methanol/ethyl acetate and finally methanol/isopropylamine/ethyl acetate (5/5/90) to afford 2.28 g (72%) of the diethyl phosphoramide as a yellow solid, m.p. 108-110°C when recrystallized from ethyl acetate.

Example 13

N-[[1-[[2-(Diethylamino)ethyl]amino]-9-oxothioxanthen-4-yl]methyl]-N-ethylformamide

(I: $R^1$=$R^2$=Et;Q=$CH_2$NEtCHO;$R^8$=H; n=2)(IV;$R^4$=Et)

A solution of 2.0 g (5.6 mmol) of 1-[[2-(diethylamino)ethyl]amino]thioxanthen-4-carboxaldehyde, N-ethylformamide (24.0 ml) and formic acid (3.0 ml, 79.5 mmol) was heated at 170°C for 4 hours. The reaction mixture was cooled, poured into water and made basic with 10% sodium hydroxide. A solid was obtained which was collected by filtration and washed with water. The solid residue was taken up in chloroform/water, and the organic layer was separated and dried over $Na_2SO_4$. The solvent was removed in vacuo and the residue was purified by radial chromatography eluting with isopropylamine/methanol/ethyl acetate (0.5/1/98.5) to afford 1.32 g (57%) of the N-ethylformamide as an orange solid, m.p. 75-77°C.

Example 14

1-[[2-(Diethylamino)ethyl]amino]-4-[(ethylamino)-methyl]thioxanthen-9-one

(I: $R^1$=$R^2$=Et; Q=$CH_2NHC_2H_5$; $R^8$=H; n=2)((V;$R^4$=Et)

By a process substantially similar to that described in Example 4, 1.29 g (92%) of the ethylamine was obtained as the dihydrochloride from 1.3 g (3.2 mmol) of the N-ethylformamide of Example 13 and 10.8 ml of 2N hydrochloric acid. The product was recrystallized from ethanol/tetrahydrofuran and melted at 160°C (dec.).

Other representative members of the genus I may be made in an analogous manner to that of Examples 1-14, substituting the appropriate 1-[[2-(dialkylamino)-ethyl]amino]- or 1-[[3-(dialkylamino)-propyl]amino]thioxanthen-4-carboxaldehyde for 1-[[2-(diethylamino)-ethyl]amino]thioxanthen-4-carboxaldehyde. The aldehydes and their precursors are described in U.S. Patent 3,294,803.

Representative examples of the invention were tested for antitumor activity in mice according to the following procedure:

The animals were pooled, implanted subcutaneously with 30 to 60 mg tumor fragments by 12-gauge trocar, and again pooled before unselective distribution to the various treatment and control groups. For early-stage treatment, chemotherapy was started within 1 to 5 days after tumor implantation while the number of cells was relatively small ($10^7$to $10^8$ cells). For advanced-stage treatment, chemotherapy was delayed until the tumor became relatively large (200 to 300 mg in size). A 300 mg tumor contains approximately 3 x $10^8$ total cells. Tumors within a given advanced-stage trial were within a 2.5-fold size range for 90% of the animals. Tumors were measured with a caliper weekly (or twice weekly for the more rapidly growing tumors). Mice were sacrificed when their tumors reached 1500 mg (i.e., before they could cause the animal discomfort). Tumor weights were estimated from two-dimensional measurements.

The treatment and control groups were measured when the control group tumors reached approximately 700 to 1200 mg in size (Median of Group). The median tumor weight of each group was determined (including zeros). The T/C value (weight of treated tumors over the weight of control tumors) in percent is an indication of antitumor effectiveness: A T/C equal to or less than 42% is considered significant antitumor activity by the Drug Evaluation Branch of the Division of Cancer Treatment (NCI). A T/C value <10% is considered to indicate highly significant antitumor activity. A body weight loss nadir (mean of group) of greater than 20% or greater than 20% drug-deaths is considered to indicate an excessively toxic dosage.

The results are shown in Table I for pancreatic ductal adenocarcinoma #03 and in Table 2 for colon adenocarcinoma #38.

TABLE 1

| Example # | T/C (%) | Weight Loss (g)* | Drug Deaths | Total Dose (mg/kg) i.v. |
|---|---|---|---|---|
| 1 | 0 | 1.6 | 0 | 1739 |
| 2 | 0 | 2.0 | 0 | 576 |
| 2 | 7 | 1.6 | 0 | 144 |
| 4 | 0 | 0.4 | 0 | 570 |
| 5 | 0 | 1.6 | 0 | 222 |
| 6 | 0 | 1.6 | 0 | 124 |
| 7 | 0 | 3.2 | 1/5 | 400 |
| 8 | 0 | 0.8 | 0 | 304 |
| 9 | 8 | 1.6 | 0 | 1395 |
| 10 | 0 | 2.4 | 0 | 540 |
| 11 | 0 | 0.8 | 0 | 855 |
| 12 | 36 | 3.2 | 0 | 1298 |
| 13 | 0 | 2.4 | 0 | 431 |

*Average body weight was 25 g.

TABLE 2

| Example # | T/C (%) | Weight Loss (g)* | Drug Deaths | Total Dose (mg/kg) i.v. |
|---|---|---|---|---|
| 2 | 0 | 2.8 | 0 | 600 |
| 5 | 11 | 2.9 | 0 | 960 |
| 6 | 0 | 5.0 | 3/7 | 132 |
| 6 | 4 | 1.7 | 1/7 | 82 |
| 7 | 16 | 0.6 | 0 | 840 |

*Average body weight was 25 g.

The compound of Example 5 was tested by intravenous infusion against a number of other tumors as shown in Table 3, and was active at 300 mg/kg p.o. against colon adenocarcinoma #38.

The compound of Example 6 was tested by bolus intravenous injection against a number of other tumors as shown in Table 4.

TABLE 3

| Tumor | Total Dosage mg/kg | Schedule | Wt. Loss at Nadir g/mouse | T/C Value in % | Tumor Free | (Days of Observation) |
|---|---|---|---|---|---|---|
| Human Adeno Squamous Lung #125 | 960 | 16 hr infusion days 7,20 | -0.4 | 16% | 1/4 | 33 |
| | | | -0.4 | 16% | 0/4 | 132 |
| | 600 | | -0.4 | 15% | 0/5 | 33 |
| | | | -0.4 | 15% | 0.5 | 132 |
| Mammary 16/C | 720 | 4 hr infusion days 1,4 | -2.0 | 18% | 0/7 | 13 |
| | 454 | | -1.2 | 16% | 0/7 | 13 |
| Mammary 16/C/Adr* | 960 | 3 hr infusion days 1,4 | -2.8 | 23% | 0/7 | 27 |
| Colon Adenocarcinoma #38 | 732 | 3-15 bolus | -2.0 | 0% | 1/5 | 100 |
| | 477 | | -2.8 | 9% | 0/5 | 100 |
| Colon Adenocarcinoma #38 | 960 | 4 hr infusion days 6,13 | -2.9 | 11% | 2/7 | 20 |
| | | | -2.9 | 11% | 1/7 | 100 |
| | 600 | | -2.0 | 26% | 0/7 | 20 |
| | | | -2.0 | 26% | 0/7 | 100 |
| Colon #51 | 720 | 3 hr infusion days 3,7 | -1.1 | 8% | 0/5 | 50 |
| | 454 | | -2.8 | 12% | 0/7 | 50 |
| Panc 03 | 222 | 3 hr infusion | -1.6 | 0 | 1/4 | 108 |

*adriamycin resistant

TABLE 4

| Tumor | Total Dosage mg/kg | Schedule | Wt. Loss at Nadir g/mouse | T/C Value in % | Tumor Free | (Days of Observation) |
|---|---|---|---|---|---|---|
| Colon Adenocarcinoma #38 | 82<br>51 | days 6-9 bolus | -1.7<br>-1.2 | 1<br>18 | 2/7<br>1/7 | 132<br>132 |
| Mammary 16/C | 82<br>51 | days 1-4 bolus | -3.0<br>-1.2 | 6<br>13 | 0/5<br>0/5 | 23<br>23 |
| Colon 51/A | 96<br>66 | days 3-6,9,11 bolus | -3.0<br>-2.3 | 14<br>28 | 0/6<br>0/6 | 32<br>32 |
| Panc 02 | 82<br>51 | days 1-4 bolus | -2.0<br>-1.2 | 23<br>42 | 0/5<br>0/5 | 28<br>28. |
| Panc 03 | 124<br>79 | days 3-4, 6-14 bolus | -1.6<br>-1.0 | 0<br>0 | 3/5<br>1/5 | 245<br>245 |

The pharmaceutical compositions of the present invention include one or more of the compounds of this invention formulated into compositions together with one or more non-toxic physiologically acceptable carriers, adjuvants or vehicles which are collectively referred to herein as carriers, for parenteral injection, for oral administration in solid or liquid form, for rectal or topical administration, and the like.

The compositions can be administered to humans and animals either orally, rectally, parenterally (intravenously, intramuscularly or subcutaneously), intracisternally, intravaginally, intraperitoneally, locally (powders, ointments or drops), or as a buccal or nasal spray.

Compositions suitable for parenteral injection may comprise physiologically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propylene glycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride and the like. Prolonged absorption of the injectable pharmaceutical form can be brought about by the use of agents delaying absorption, for example, aluminum monostearate and gelatin.

If desired, and for more effective distribution, the compounds can be incorporated into slow release or targeted delivery systems such as polymer matrices, liposomes, and microspheres. They may be sterilized, for example, by filtration through a bacteria-retaining filter, or by incorporating sterilizing agents in the form of sterile solid compositions which can be dissolved in sterile water, or some other sterile injectable medium immediately before use.

Solid dosage forms for oral administration include capsules, tablets, pills, powders and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol and silicic acid, (b) binders, as for example, carboxymethylcellulose, alignates, gelatin, polyvinylpyrrolidone, sucrose and acacia, (c) humectants, as for example, glycerol, (d) disintegrating agents, as for example, agar-agar, calcium carbonate, potato or tapioca starch, alginic acid, certain complex silicates and sodium carbonate, (e) solution retarders, as for example, paraffin, (f) absorption accelerators, as for example, quaternary ammonium compounds, (g) wetting agents, as for example, cetyl alcohol and glycerol monostearate, (h) adsorbents, as for example, kaolin and bentonite, and (i) lubricants, as for example, talc, calcium stearate, magnesium stearate, solid polyethylene glycols, sodium lauryl sulfate or mixtures thereof. In the case of capsules, tablets and pills, the dosage forms may also comprise buffering agents.

The percentage of active component in the composition and method for treating tumors or cancer can be varied so that a suitable dosage is obtained. The dosage administered to a particular patient is variable depending upon the clinician's judgement using as the criteria: the route of administration, the duration of treatment, the size and condition of the patient, the potency of the active component, and the patient's response thereto. An effective dosage amount of active component can thus readily be determined by the clinician considering all criteria and utilizing his best judgement on the patient's behalf.

## Claims

### Claims for the following Contracting States : AT, BE, CH, LI, DE, DK, FR, GB, IT, LU, MC, NL, PT, SE

1. A compound of formula

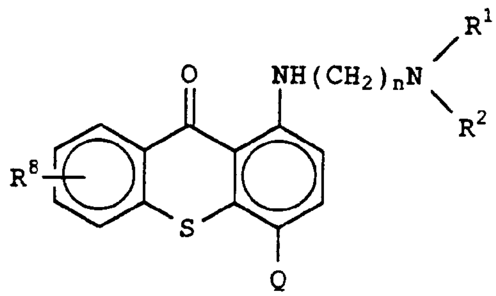

wherein n is 2 or 3;

| | |
|---|---|
| $R^1$ and $R^2$ | are independently $C_1$-$C_4$-alkyl; |
| Q | is a residue chosen from the group consisting of $CH_2NHR^3$, $CH_2N(R^4)SO_2R^7$, $CH_2NHCHO$, CH=N-Ar, $C(O)NR^5R^6$, $CH_2N(R^4)C(O)R^7$, $CH_2N(C_2H_5)CHO$, and $CH_2N(R^4)P(O)(O$-$C_1$-$C_4$-alkyl$)_2$; |
| $R^3$ | is hydrogen or $C_1$-$C_4$-alkyl; |
| $R^4$ | is hydrogen or $C_1$-$C_4$-alkyl; |
| $R^5$ | is hydrogen, $C_1$-$C_4$-alkyl or Ar; |
| $R^6$ | is hydrogen or $C_1$-$C_4$-alkyl; |
| $R^7$ | is $C_1$-$C_4$-alkyl, or Ar; |
| $R^8$ | is hydrogen, $C_1$-$C_4$-alkyl, $C_1$-$C_4$-alkoxy, or halogen; and |
| Ar | is phenyl or phenyl substituted with methyl, methoxy, halogen or nitro; |

or a pharmaceutically acceptable acid-addition salt or solvate thereof.

**2.** A compound according to claim 1 wherein:

| | |
|---|---|
| Q | is a residue chosen from the group consisting of $CH_2NHR^3$, $CH_2N(R^4)SO_2R^7$, $CH_2NHCHO$, CH=N-Ar and $C(O)NR^5R^6$,; |
| $R^3$ | is hydrogen or methyl; and |
| $R^7$ | is $C_1$-$C_4$-alkyl. |

**3.** A compound according to claim 2 wherein n is 2 and both of $R^1$ and $R^2$ are ethyl, and $R^8$ is hydrogen.

**4.** A compound according to claim 3 wherein Q is a residue chosen from the group consisting of $CH_2NHR^3$, $CH_2N(R^4)SO_2R^7$ and $CH_2NHCHO$.

**5.** A compound according to claim 4 wherein Q is $CH_2NHR^3$.

**6.** 1-[[2-(Diethylamino)ethyl]amino]-4-[(methylamino)methyl]-thioxanthen-9-one.

**7.** A compound according to claim 1 wherein Q is a residue chosen from the group consisting of $CH_2N(R^4)SO_2R^7$, $CH_2N(R^4)C(O)R^7$, $CH_2N(C_2H_5)CHO$ and $CH_2N(R^4)P(O)(O$-$C_1$-$C_4$-alkyl$)_2$.

**8.** A compound according to claim 7 wherein n is 2 and both of $R^1$ and $R^2$ are ethyl, and $R^8$ is hydrogen.

**9.** A compound according to claim 8 wherein Q is $CH_2N(R^4)C(O)R^7$.

**10.** A compound according to claim 8 wherein Q is $CH_2N(R^4)SO_2R^7$.

**11.** N-[[1-[[2-(Diethylamino)ethyl]amino]-9-oxothioxanthen-4-yl]methyl]methanesulfonamide.

**12.** A pharmaceutical composition which comprises a compound of claim 1 and a pharmaceutically acceptable carrier or diluent.

**13.** A pharmaceutical composition which comprises a compound of claim 6 and a pharmaceutically acceptable carrier or diluent.

**14.** A pharmaceutical composition which comprises a compound of claim 11 and a pharmaceutically acceptable carrier or diluent.

**15.** The use of a compound of claim 1 for the preparation of a medicament for treating a tumor in a mammal.

**16.** The use of a compound of claim 6 for the preparation of a medicament for treating a tumor in a mammal.

**17.** The use of a compound of claim 11 for the preparation of a medicament for treating a tumor in a mammal.

**18.** The use of a composition of claim 12 for the preparation of a medicament for treating cancer in a mammal.

**19.** The use of a composition of claim 13 for the preparation of a medicament for treating cancer in a mammal.

**20.** The use of a composition of claim 14 for the preparation of a medicament for treating cancer in a mammal.

**Claims for the following Contracting States : ES, GR**

**1.** A process for preparing a compound having the formula

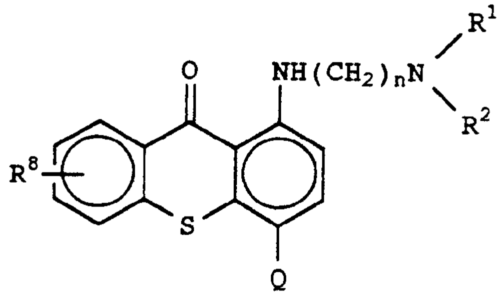

wherein n is 2 or 3;

| | |
|---|---|
| $R^1$ and $R^2$ | are independently $C_1$-$C_4$-alkyl; |
| Q | is a residue chosen from the group consisting of $CH_2NHR^3$, $CH_2N(R^4)SO_2R^7$, $CH_2NHCHO$, CH=N-Ar, $C(O)NR^5R^6$, $CH_2N(R^4)C(O)R^7$, $CH_2N(C_2H_5)CHO$, and $CH_2N(R^4)P(O)(O$-$C_1$-$C_4$-alkyl$)_2$; |
| $R^3$ | is hydrogen or $C_1$-$C_4$-alkyl; |
| $R^4$ | is hydrogen or $C_1$-$C_4$-alkyl; |
| $R^5$ | is hydrogen, $C_1$-$C_4$-alkyl or Ar; |
| $R^6$ | is hydrogen or $C_1$-$C_4$-alkyl; |
| $R^7$ | is $C_1$-$C_4$-alkyl, or Ar; |
| $R^8$ | is hydrogen, $C_1$-$C_4$-alkyl, $C_1$-$C_4$-alkoxy, or halogen; and |
| Ar | is phenyl or phenyl substituted with methyl, methoxy, halogen or nitro; |

or a pharmaceutically acceptable acid-addition salt or solvate thereof, which comprises

1) reacting a compound of formula II

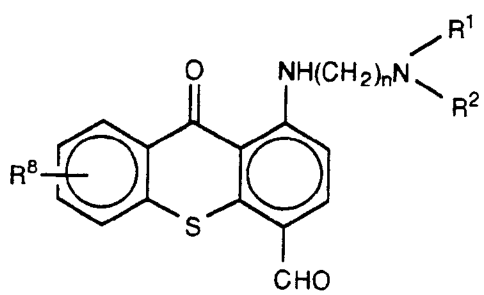

II

with

(a) a compound of formula $ArNH_2$, to prepare a corresponding compound of formula I wherein Q is CH=N-Ar;

(b) formamide or N-ethylformamide to prepare a corresponding compound of formula I wherein Q

is $CH_2NHCHO$ or $CH_2N(C_2H_5)CHO$; or
(c) hydroxylamine to prepare a compound of formula VII

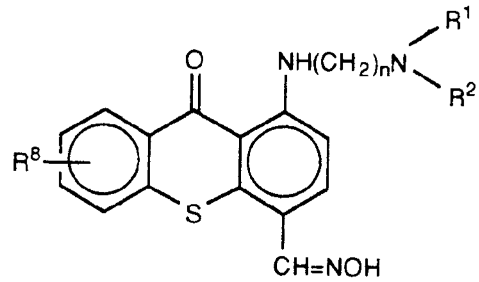

VII

2) reacting said compound of formula VII with an agent capable of dehydrating an oxime to prepare a compound of formula VIII

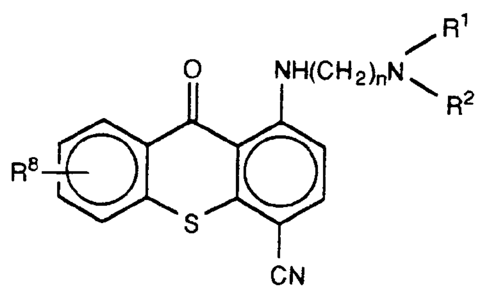

VIII

and reacting said latter compound of formula VIII with
(a) an aqueous acid to prepare a corresponding compound of formula I wherein Q is $C(O)NH_2$, or
(b) an aqueous base to prepare a compound of formula I wherein Q is $CO_2H$,
and thereafter reacting said latter compound with an amine to prepare a compound of formula I wherein Q is $C(O)NR^5R^6$;
3) reacting a compound of formula

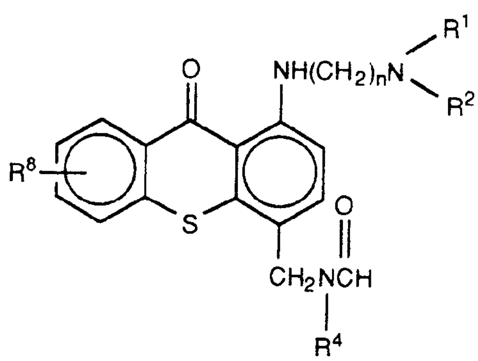

where $R^4$=$R^3$, with an aqueous acid to prepare a corresponding compound of formula I wherein Q is $CH_2NHR^3$;
4) reacting said latter compound of formula I where Q is $CH_2NHR^3$, where $R^3$=$R^4$, with a sulfonyl halide of formula , $R^7SO_2X$, to prepare a corresponding compound of formula I wherein Q is $CH_2N(R^4)SO_2R^7$; and
5) reacting said compound of formula I wherein Q is $CH_2NHR^3$, where $R^3$=$R^4$, with
(a) an acid halide of formula $R^7C(O)X$, to prepare a corresponding compound of formula I wherein Q is $CH_2N(R^4)C(O)R^7$; or

(b) with a di-$C_1$-$C_4$-alkyl phosphorochloridate of formula, $(C_1\text{-}C_4\text{-alkyl-O})_2\text{-P(O)Cl}$, to prepare a corresponding compound of formula I wherein Q is $CH_2N(R^4)P(O)\text{-}(O\text{-}C_1\text{-}C_4\text{-alkyl})_2$.

**2.** A process according to claim 1 wherein:
Q is a residue chosen from the group consisting of $CH_2NHR^3$, $CH_2N(R^4)SO_2R^7$, $CH_2NHCHO$, CH=N-Ar and $C(O)NR^5R^6$;
$R^3$ is hydrogen or methyl; and
$R^7$ is $C_1$-$C_4$-alkyl.

**3.** A process according to claim 2 wherein n is 2 and both of $R^1$ and $R^2$ are ethyl, and $R^8$ is hydrogen.

**4.** A process according to claim 3 wherein Q is a residue chosen from the group consisting of $CH_2NHR^3$, $CH_2N(R^4)SO_2R^7$, and $CH_2NHCHO$.

**5.** A process according to claim 4 for preparing 1-[[2-(Diethylamino)ethyl]amino]-4-[(methylamino)methyl]-thioxanthen-9-one.

**6.** A process according to claim 1 wherein Q is a residue chosen from the group consisting of $CH_2N(R^4)SO_2R^7$, $CH_2N(R^4)C(O)R^7$, $CH_2N(C_2H_5)CHO$ and $CH_2N(R^4)P(O)(O\text{-}C_1\text{-}C_4\text{-alkyl})_2$.

**7.** A process according to claim 6 wherein n is 2 and both of $R^1$ and $R^2$ are ethyl, and $R^8$ is hydrogen.

**8.** A process according to claim 7 wherein Q is a residue chosen from the group consisting of $CH_2N(R^4)C(O)R^7$ and $CH_2N(R^4)SO_2R^7$.

**9.** A process according to claim 8 for preparing N-[[1-[[2-(Diethylamino)ethyl]amino]-9-oxothioxanthen-4-yl]methyl]methanesulfonamide.

## Patentansprüche

### Patentansprüche für folgende Vertragsstaaten : AT, BE, CH, DE, DK, FR, GB, IT, LI, LU, MC, NL, PT, SE

**1.** Verbindung der Formel

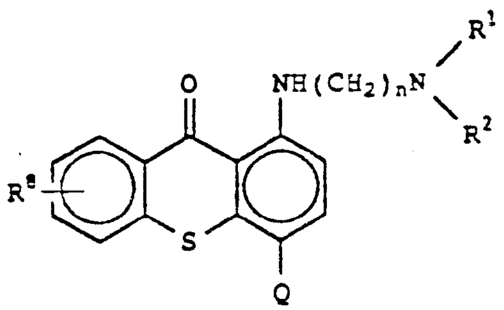

worin bedeuten: n gleich 2 oder 3;
$R^1$ und $R^2$ unabhängig voneinander $C_1$-$C_4$-Alkyl;
Q einen Rest, ausgewählt aus der Gruppe bestehend aus $-CH_2NHR^3$, $-CH_2NH(R^4)SO_2R^7$, $CH_2NHCHO$, -CH=N-Ar, $-C(O)NR^5R^6$, $-CH_2N(R^4)C(O)R^7$, $-CH_2N(C_2H_5)CHO$, und $-CH_2N(R^4)P(O)\ (O\text{-}C_1\text{-}C_4\text{-Alkyl})_2$;
$R^3$ Wasserstoff oder $C_1$-$C_4$-Alkyl;
$R^4$ Wasserstoff oder $C_1$-$C_4$-Alkyl;
$R^5$ Wasserstoff, $C_1$-$C_4$-Alkyl oder Ar;
$R^6$ Wasserstoff oder $C_1$-$C_4$-Alkyl;
$R^7$ $C_1$-$C_4$-Alkyl oder Ar;
$R^8$ Wasserstoff, $C_1$-$C_4$-Alkyl, $C_1$-$C_4$-Alkoxy oder Halogen, und
Ar Phenyl oder Phenyl substituiert durch Methyl, Methoxy, Halogen oder Nitro;

oder ein pharmazeutisch akzeptierbares Säure-Additionssalz oder ein Solvat hiervon.

2. Verbindung nach Anspruch 1, in der:
Q ein Rest, ausgewählt aus der Gruppe bestehend aus $-CH_2NHR^3$, $-CH_2N(R^4)SO_2R^7$, $-CH_2NHCHO$, -CH=N-Ar und $-C(O)NR^5R^6$ ist;
$R^3$ für Wasserstoff oder Methyl steht; und
$R^7$ $C_1$-$C_4$-Alkyl ist.

3. Verbindung nach Anspruch 2, worin n für 2 steht und sowohl $R^1$ als auch $R^2$ für Ethyl stehen, und $R^8$ Wasserstoff ist.

4. Verbindung nach Anspruch 3, worin Q ein Rest ist, ausgewählt aus der Gruppe bestehend aus $-CH_2NHR^3$, $-CH_2N(R^4)SO_2R^7$ und $-CH_2NHCHO$.

5. Verbindung nach Anspruch 4, worin Q für $-CH_2NHR^3$ steht.

6. 1-[[2-(Diethylamino)ethyl]amino]-4-[(methylamino)methyl]-thioxanthen-9-on.

7. Verbindung nach Anspruch 1, worin Q ein Rest ist, ausgewählt aus der Gruppe bestehend aus $-CH_2N(R^4)SO_2R^7$, $-CH_2N(R^4)C(O)R^7$, $-CH_2N(C_2H_5)CHO$ und $-CH_2N(R^4)P(O)$ $(O$-$C_1$-$C_4$-Alkyl$)_2$.

8. Verbindung nach Anspruch 7, worin n für 2 steht und sowohl $R^1$ als auch $R^2$ Ethyl bedeuten und $R^8$ Wasserstoff ist.

9. Verbindung nach Anspruch 8, worin Q für $-CH_2N(R^4)C(O)R^7$ steht.

10. Verbindung nach Anspruch 8, worin Q für $-CH_2N(R^4)SO_2R^7$ steht.

11. N-[[1-[[2-(Diethylamino)ethyl]amino]-9-oxothioxanthen-4-yl]methyl]methansulfonamid.

12. Pharmazeutische Zusammensetzung mit einer Verbindung nach Anspruch 1 und einem pharmazeutisch verträglichen Träger oder Verdünnungsmittel.

13. Pharmazeutische Zusammensetzung mit einer Verbindung nach Anspruch 6 und einem pharmazeutisch verträglichen Träger oder Verdünnungsmittel.

14. Pharmazeutische Zusammensetzung mit einer Verbindung nach Anspruch 11 und einem pharmazeutisch verträglichen Träger oder Verdünnungsmittel.

15. Verwendung einer Verbindung nach Anspruch 1 zur Herstellung eines Medikamentes für die Behandlung eines Tumors in einem Säuger.

16. Verwendung einer Verbindung nach Anspruch 6 für die Herstellung eines Medikamentes zur Behandlung eines Tumors in einem Säuger.

17. Verwendung einer Verbindung nach Anspruch 11 für die Herstellung eines Medikamentes zur Behandlung eines Tumors in einem Säuger.

18. Verwendung einer Zusammensetzung nach Anspruch 12 für die Herstellung eines Medikamentes zur Behandlung von Krebs in einem Säuger.

19. Verwendung einer Zusammensetzung nach Anspruch 13 zur Herstellung eines Medikamentes zur Behandlung von Krebs in einem Säuger.

20. Verwendung einer Zusammensetzung nach Anspruch 14 für die Herstellung eines Medikamentes zur Behandlung von Krebs in einem Säuger.

**Patentansprüche für folgende Vertragsstaaten : ES, GR**

1. Verfahren zur Herstellung einer Verbindung der Formel:

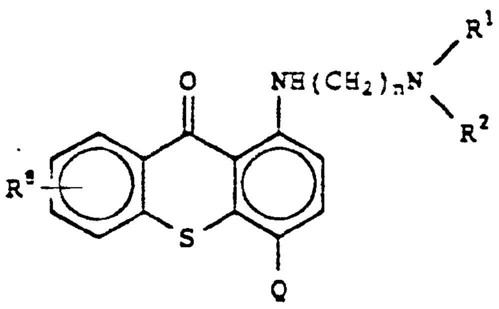

worin bedeuten: n gleich 2 oder 3;
$R^1$ und $R^2$ unabhängig voneinander $C_1$-$C_4$-Alkyl;
Q einen Rest, ausgewählt aus der Gruppe bestehend aus $-CH_2NHR^3$, $CH_2N(R^4)SO_2R^7$, $-CH_2NHCHO$, -CH=N-Ar, $-C(O)NR^5R^6$, $-CH_2N(R^4)C(O)R^7$, $-CH_2N(C_2H_5)CHO$, und $-CH_2N(R^4)P(O)$ $(O-C_1-C_4\text{-Alkyl})_2$;
$R^3$ Wasserstoff oder $C_1$-$C_4$-Alkyl;
$R^4$ Wasserstoff oder $C_1$-$C_4$-Alkyl;
$R^5$ Wasserstoff, $C_1$-$C_4$-Alkyl oder Ar;
$R^6$ Wasserstoff oder $C_1$-$C_4$-Alkyl;
$R^7$ $C_1$-$C_4$-Alkyl oder Ar;
$R^8$ Wasserstoff, $C_1$-$C_4$-Alkyl, $C_1$-$C_4$-Alkoxy oder Halogen, und
Ar Phenyl oder Phenyl substituiert durch Methyl, Methoxy, Halogen oder Nitro;
oder ein pharmazeutisch akzeptierbares Säure-Additionssalz oder ein Solvat hiervon, bei dem man

1) eine Verbindung der Formel II

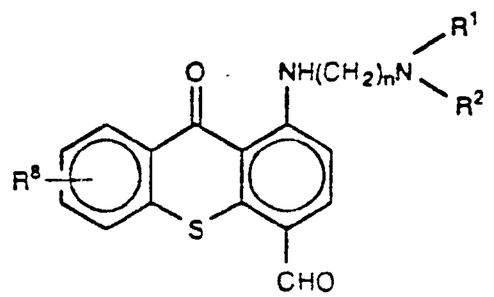

II

umsetzt mit

(a) einer Verbindung der Formel $ArNH_2$, unter Herstellung einer entsprechenden Verbindung der Formel I, worin Q für -CH=N-Ar steht;
(b) Formamid oder N-Ethylformamid unter Herstellung einer entsprechenden Verbindung der Formel I, worin Q steht für $-CH_2NHCHO$ oder $-CH_2N(C_2H_5)CHO$; oder
(c) Hydroxylamin, unter Herstellung einer Verbindung der Formel VII

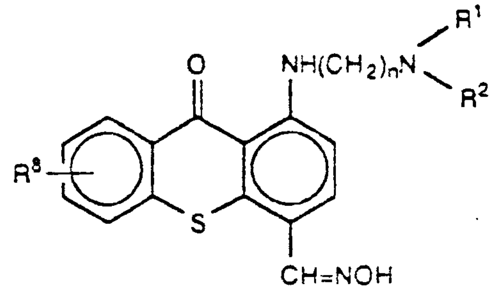

VII

2) die Verbindung der Formel VII umsetzt mit einem Mittel, das dazu befähigt ist, ein Oxim zu dehydratisieren, unter Herstellung einer Verbindung der Formel VIII

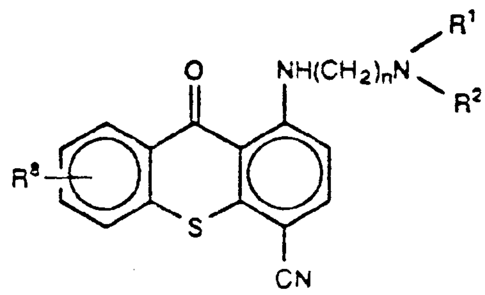

VIII

und Umsetzung dieser Verbindung der Formel VIII mit

(a) einer wäßrigen Säure unter Herstellung einer entsprechenden Verbindung der Formel I, worin Q steht für $-C(O)NH_2$, oder

(b) einer wäßrigen Base, unter Herstellung einer Verbindung der Formel I, worin Q steht für $-CO_2H$, worauf man die letztere Verbindung mit einem Amin umsetzt, unter Herstellung einer Verbindung der Formel I, worin Q steht für $-C(O)NR^5R^6$;

3) Umsetzung einer Verbindung der Formel

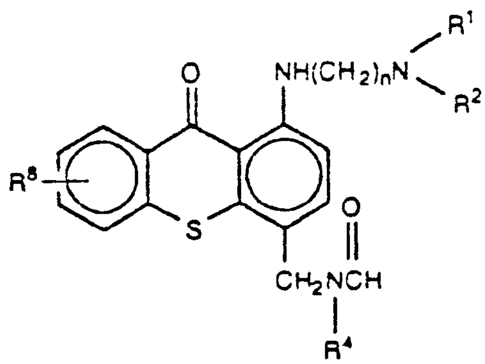

worin $R^4$=$R^3$ ist, mit einer wäßrigen Säure, unter Herstellung einer entsprechenden Verbindung der Formel I, worin Q steht für $-CH_2NHR^3$;

4) Umsetzung der letzteren Verbindung der Formel I, worin Q steht für $-CH_2NHR^3$, worin $R^3$=$R^4$ ist, mit einem Sulfonylhalogenid der Formel $R^7SO_2X$, unter Herstellung einer entsprechenden Verbindung der Formel I, worin Q steht für $-CH_2N(R^4)SO_2R^7$; und

5) Umsetzung der Verbindung der Formel I, worin Q für $-CH_2NHR^3$ steht, worin $R^3$=$R^4$ ist, mit

(a) einem Säurehalogenid der Formel $R^7C(O)X$, unter Herstellung einer entsprechenden Verbindung der Formel I, worin Q steht für $-CH_2N(R^4)C(O)R^7$; oder

(b) mit einem Di-$C_1$-$C_4$-Alkylphosphorochloridat der Formel ($C_1$-$C_4$-Alkyl-O)$_2$-P(O)Cl, unter Herstellung einer entsprechenden Verbindung der Formel I, worin Q steht für $-CH_2N(R^4)P(O)-(O-C_1-C_4\text{-Alkyl})_2$.

2. Verfahren nach Anspruch 1, worin:
Q ein Rest ist, ausgewählt aus der Gruppe bestehend aus: $-CH_2NHR^3$, $-CH_2N(R^4)SO_2R^7$, $CH_2NHCHO$, -CH=N-Ar und $-C(O)NR^5R^6$;
$R^3$ für Wasserstoff oder Methyl steht; und
$R^7$ $C_1$-$C_4$-Alkyl ist.

3. Verfahren nach Anspruch 2, worin n gleich 2 ist und sowohl $R^1$ als auch $R^2$ für Ethyl stehen und $R^8$ Wasserstoff darstellt.

4. Verfahren nach Anspruch 3, worin Q ein Rest ist, ausgewählt aus der Gruppe bestehend aus $-CH_2NHR^7$,

$-CH_2N(R^4)SO_2R^7$ und $-CH_2NHCHO$.

**5.** Verfahren nach Anspruch 4 zur Herstellung von 1-[[2-(Diethylamino)ethyl]amino]-4-[(methylamino)methyl]-thioxanthen-9-on.

**6.** Verfahren nach Anspruch 1, worin Q ein Rest ist, ausgewählt aus der Gruppe bestehend aus $-CH_2N(R^4)SO_2R^7$, $-CH_2N(R^4)C(O)R^7$, $-CH_2N(C_2H_5)CHO$ und $-CH_2N(R^4)P(O)(O-C_1-C_4\text{-Alkyl})$ .

**7.** Verfahren nach Anspruch 6, worin n gleich 2 ist und sowohl $R^1$ wie auch $R^2$ für Ethyl stehen und $R^8$ Wasserstoff darstellt.

**8.** Verfahren nach Anspruch 7, in dem Q ein Rest ist, ausgewählt aus der Gruppe, bestehend aus $-CH_2N(R^4)C(O)R^7$ und $-CH_2N(R^4)SO_2R^7$.

**9.** Verfahren nach Anspruch 8 zur Herstellung von N-[[1-[[2-(Diethylamino)ethyl]amino]-9-oxothioxanthen-4-yl]methyl]-methansulfonamid.

## Revendications

**Revendications pour les Etats contractants suivants : AT, BE, CH, LI, DE, DK, FR, GB, IT, LU, MC, NL, PT, SE**

**1.** Composé de formule :

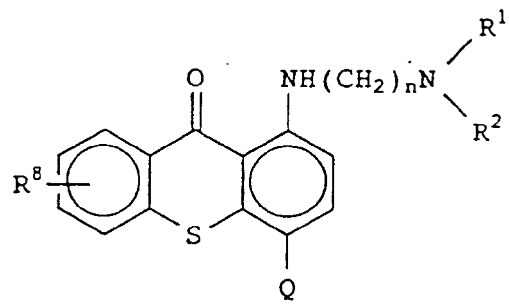

dans laquelle n est 2 ou 3 ;

| | |
|---|---|
| $R^1$ et $R^2$ | sont indépendamment un alkyle en $C_1$-$C_4$ ; |
| Q | est un résidu choisi dans le groupe formé par $CH_2NHR^3$, $CH_2N(R^4)SO_2R^7$, $CH_2NHCHO$, $CH{=}N\text{-}Ar$, $C(O)NR^5R^6$, $CH_2N(R^4)C(O)R^7$, $CH_2N(C_2H_5)CHO$ et $CH_2N(R^4)P(O)(O\text{-alkyle en } C_1\text{-}C_4)_2$ ; |
| $R^3$ | est l'hydrogène ou un alkyle en $C_1$-$C_4$ ; |
| $R^4$ | est l'hydrogène ou un alkyle en $C_1$-$C_4$ ; |
| $R^5$ | est l'hydrogène, un alkyle en $C_1$-$C_4$ ou Ar ; |
| $R^6$ | est l'hydrogène ou un alkyle en $C_1$-$C_4$ ; |
| $R^7$ | est un alkyle en $C_1$-$C_4$ ou Ar ; |
| $R^8$ | est l'hydrogène, un alkyle en $C_1$-$C_4$, un alcoxy en $C_1$-$C_4$ ou un halogène ; et |
| Ar | est un phényle ou un phényle substitué par méthyle, méthoxy, halogène ou nitro ; |

ou un sel d'addition d'acide ou solvate pharmaceutiquement acceptable de ce composé.

**2.** Composé selon la revendication 1, dans lequel :

Q est un résidu choisi dans le groupe formé par $CH_2NHR^3$, $CH_2N(R^4)SO_2R^7$, $CH_2NHCHO$, $CH{=}N\text{-}Ar$ et $C(O)NR^5R^6$ ;

$R^3$ est l'hydrogène ou un méthyle ; et

$R^7$ est un alkyle en $C_1$-$C_4$.

**3.** Composé selon la revendication 2, dans lequel n est 2 et $R^1$ et $R^2$ sont l'un et l'autre un éthyle, et $R^8$ est l'hydrogène.

4. Composé selon la revendication 3, dans lequel Q est un résidu choisi dans le groupe formé par $CH_2NHR^3$, $CH_2N(R^4)SO_2R^7$ et $CH_2NHCHO$.

5. Composé selon la revendication 4, dans lequel Q est $CH_2NHR^3$.

6. 1-[[2-(diéthylamino)éthyl]amino]-4-[(méthylamino)méthyl]-thioxanthén-9-one.

7. Composé selon la revendication 1, dans lequel Q est un résidu choisi dans le groupe formé par $CH_2N(R^4)SO_2R^7$, $CH_2N(R^4)C(O)R^7$, $CH_2N(C_2H_5)CHO$ et $CH_2N(R^4)P(O)$(O-alkyle en $C_1$-$C_4$)$_2$.

8. Composé selon la revendication 7, dans lequel n est 2 et $R^1$ et $R^2$ sont l'un et l'autre un éthyle, et $R^8$ est l'hydrogène.

9. Composé selon la revendication 8, dans lequel Q est $CH_2N(R^4)C(O)R^7$.

10. Composé selon la revendication 8, dans lequel Q est $CH_2N(R^4)SO_2R^7$.

11. N-[[1-[[2-(diéthylamino)éthyl]amino]-9-oxothioxanthén-4-yl]méthyl]méthanesulfonamide.

12. Composition pharmaceutique qui comprend un composé selon la revendication 1 et un véhicule ou diluant pharmaceutiquement acceptable.

13. Composition pharmaceutique qui comprend un composé selon la revendication 6 et un véhicule ou diluant pharmaceutiquement acceptable.

14. Composition pharmaceutique qui comprend un composé selon la revendication 11 et un véhicule ou diluant pharmaceutiquement acceptable.

15. Utilisation d'un composé selon la revendication 1 pour la préparation d'un médicament pour traiter une tumeur chez un mammifère.

16. Utilisation d'un composé selon la revendication 6 pour la préparation d'un médicament pour traiter une tumeur chez un mammifère.

17. Utilisation d'un composé selon la revendication 11 pour la préparation d'un médicament pour traiter une tumeur chez un mammifère.

18. Utilisation d'une composition selon la revendication 12 pour la préparation d'un médicament pour le traitement du cancer chez un mammifère.

19. Utilisation d'une composition selon la revendication 13 pour la préparation d'un médicament pour le traitement du cancer chez un mammifère.

20. Utilisation d'une composition selon la revendication 14 pour la préparation d'un médicament pour le traitement du cancer chez un mammifère.

**Revendications pour les Etats contractants suivants : ES, GR**

1. Procédé de préparation d'un composé de formule :

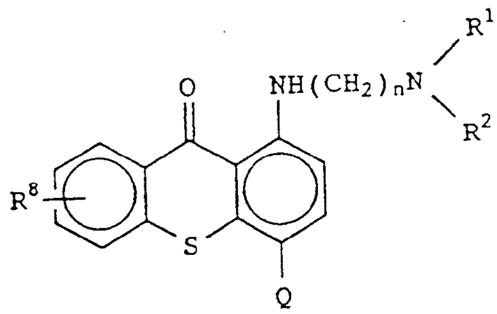

dans laquelle n est 2 ou 3 ;

| | |
|---|---|
| $R^1$ et $R^2$ | sont indépendamment un alkyle en $C_1$-$C_4$ ; |
| Q | est un résidu choisi dans le groupe formé par $CH_2NHR^3$, $CH_2N(R^4)SO_2R^7$, $CH_2NHCHO$, CH=N-Ar, $C(O)NR^5R^6$, $CH_2N(R^4)O(O)R^7$, $CH_2N(C_2H_5)CHO$ et $CH_2N(R^4)P(O)$(O-alkyle en $C_1$-$C_4)_2$ ; |
| $R^3$ | est l'hydrogène ou un alkyle en $C_1$-$C_4$ ; |
| $R^4$ | est l'hydrogène ou un alkyle en $C_1$-$C_4$ ; |
| $R^5$ | est l'hydrogène, un alkyle en $C_1$-$C_4$ ou Ar ; |
| $R^6$ | est l'hydrogène ou un alkyle en $C_1$-$C_4$ ; |
| $R^7$ | est un alkyle en $C_1$-$C_4$ ou Ar ; |
| $R^8$ | est l'hydrogène, un alkyle en $C_1$-$C_4$, un alcoxy en $C_1$-$C_4$ ou un halogène ; et |
| Ar | est un phényle ou un phényle substitué par méthyle, méthoxy, halogène ou nitro ; |

ou d'un sel d'addition d'acide ou solvate pharmaceutiquement acceptable de ce composé, qui comprend:

1) la réaction d'un composé de formule II :

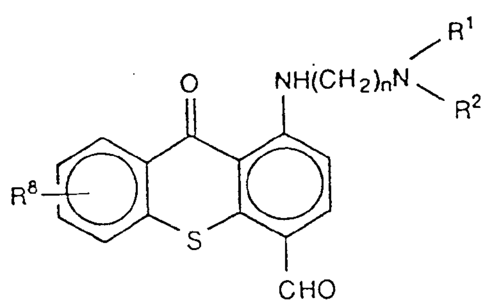

II

avec

(a) un composé de formule $ArNH_2$ pour préparer un composé correspondant de formule I où Q est CH=N-Ar ;

(b) le formamide ou le N-éthylformamide pour préparer un composé correspondant de formule I où Q est $CH_2NHCHO$ ou $CH_2N(C_2H_5)CHO$ ; ou

(c) l'hydroxylamine pour préparer un composé de formule VII :

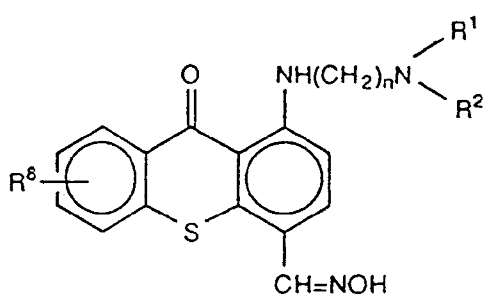

VII

2) la réaction dudit composé de formule VII avec un agent capable de déshydrater une oxime pour préparer un composé de formule VIII :

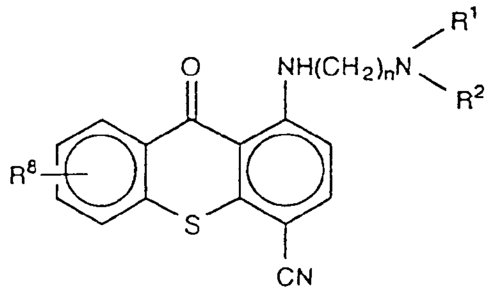

VIII

et la réaction de ce dernier composé de formule VIII avec

(a) un acide aqueux pour préparer un composé correspondant de formule I où Q est $C(O)NH_2$, ou

(b) une base aqueuse pour préparer un composé de formule I où Q est $CO_2H$,

puis la réaction de ce dernier composé avec une amine pour préparer un composé de formule I où Q est $C(O)NR^5R^6$ ;

3) la réaction d'un composé de formule :

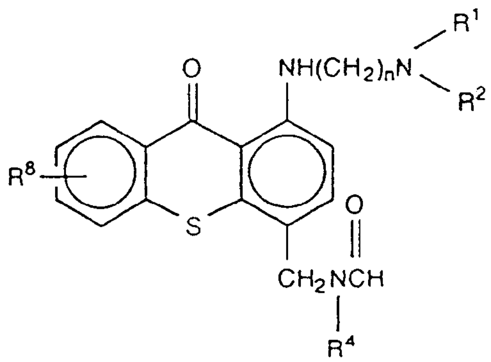

dans laquelle $R^4$=$R^3$, avec un acide aqueux pour préparer un composé correspondant de formule I où Q est $CH_2NHR^3$ ;

4) la réaction de ce dernier composé de formule I, dans laquelle Q est $CH_2NHR^3$, où $R^3$=$R^4$, avec un halogénure de sulfonyle de formule $R^7SO_2X$ pour préparer un composé correspondant de formule I où Q est $CH_2N(R^4)SO_2R^7$ ; et

5) la réaction dudit composé de formule I, dans laquelle Q est $CH_2NHR^3$, où $R^3$=$R^4$, avec

(a) un halogénure d'acide de formule $R^7C(O)X$ pour préparer un composé correspondant de formule I où Q est $CH_2N(R^4)C(O)R^7$ ; ou

(b) avec un chlorophosphate de dialkyle en $C_1$-$C_4$ de formule (alkyle en $C_1$-$C_4$-O)$_2$-P(O)Cl pour préparer un composé correspondant de formule I où Q est $CH_2N(R^4)P(O)$(O-alkyle en $C_1$-$C_4$)$_2$.

**2.** Procédé selon la revendication 1, dans lequel :

Q est un résidu choisi dans le groupe formé par $CH_2NHR^3$, $CH_2N(R^4)SO_2R^7$, $CH_2NHCHO$, CH=N-Ar et $C(O)NR^5R^6$ ;

$R^3$ est l'hydrogène ou un méthyle ; et

$R^7$ est un alkyle en $C_1$-$C_4$.

**3.** Procédé selon la revendication 2, dans lequel n est 2 et $R^1$ et $R^2$ sont l'un et l'autre un éthyle, et $R^8$ est l'hydrogène.

**4.** Procédé selon la revendication 3, dans lequel Q est un résidu choisi dans le groupe formé par $CH_2NHR^3$, $CH_2N(R^4)SO_2R^7$ et $CH_2NHCHO$.

**5.** Procédé selon la revendication 4 pour préparer la 1-[[2-(diéthylamino)éthyl]amino]-4-[(méthylamino)méthyl]-thioxanthén-9-one.

**6.** Procédé selon la revendication 1, dans lequel Q est un résidu choisi dans le groupe formé par $CH_2N(R^4)SO_2R^7$, $CH_2N(R^4)C(O)R^7$, $CH_2N(C_2H_5)CHO$ et $CH_2N(R^4)P(O)(O\text{-alkyle en } C_1\text{-}C_4)_2$.

**7.** Procédé selon la revendication 6, dans lequel n est 2 et $R^1$ et $R^2$ sont l'un et l'autre un éthyle, et $R^8$ est l'hydrogène.

**8.** Procédé selon la revendication 7, dans lequel Q est un résidu choisi dans le groupe formé par $CH_2N(R^4)C(O)R^7$ et $CH_2N(R^4)SO_2R^7$.

**9.** Procédé selon la revendication 8 pour préparer le N-[[1-[[2-(diéthylamino)éthyl]amino]-9-oxothioxanthén-4-yl]méthyl]méthanesulfonamide.